# EUROPEAN PATENT SPECIFICATION

(11) **EP 3 167 932 B1**
(45) Date of publication and mention of the grant of the patent: **22.05.2019**
(21) Application number: 15819640.2
(22) Date of filing: 01.04.2015
(51) Int. Cl.: A61N 5/067, A61B 18/20, A61N 5/06, A61B 18/00

(54) **DEVICE FOR THE TREATMENT OF A TISSUE BY MEANS OF LASER RADIATION**
VORRICHTUNG ZUR BEHANDLUNG VON GEWEBE MITTELS LASERSTRAHLUNG
DISPOSITIF POUR LE TRAITEMENT D'UN TISSU PAR RAYONNEMENT LASER

(30) Priority: 11.07.2014 ES 201431053
(43) Date of publication of application: 17.05.2017
(73) Proprietor: INTERMEDIC ARFRAN, S.A., 08290 Cerdanyola del Vallès (ES)
(72) Inventor: ARCUSA VILLACAMPA, Francisco Javier, 08290 Cerdanyola del Vallès (Barcelona) (ES)
(74) Representative: Ponti & Partners, S.L.P
(86) International application number: PCT/ES2015/070265
(87) International publication number: WO 2016/005622

(56) References cited:
- EP-A1- 2 476 460
- WO-A1-2011/096006
- WO-A2-2012/092508
- CN-U- 203 195 763
- KR-B1- 101 363 550
- KR-B1- 101 406 871

## Description

### Object of the Invention

More specifically, the invention refers to an electro-mechanical device specially designed so that in combination with a laser generator it can be used to treat the inner tissue of the vagina and, as a consequence of said treatment, retract the pelvic floor so that the bladder is lifted and due to its change in angle, slight or moderate urine leakage is resolved or improved.

With the same device and laser generator, it is also possible to treat the inner surface of the vagina, reducing possible laxity arising from childbirth and aging.

### State of the Art

The treatment of loss of urine, also called urinary incontinence, is a problem associated with human beings, and specifically women, and with diagnosis, it is possible to correct it through various types of treatment, some of which are surgical (placement of a mesh) and others, non-invasive (rehabilitation).

"Urinary incontinence" is a generic colloquial term that includes various types and degrees of loss of urine. Some specialists have classified it as stress incontinence, which is to say, incontinence experienced when laughing, coughing, or sneezing, or when making any kind of physical effort; urge incontinence, when women have an intense, immediate need to urinate, and the urine leaks out even before getting to the toilet; mixed incontinence, which is a combination of the two previous kinds; and finally, overflow incontinence, when there are constant accidental leaks of small amounts of urine.

The causes of urinary incontinence in women are diverse, some of them are short-term, and thus easier to solve, while others are a long-term consequence of a lack of support of the bladder in the pelvic floor. The short-term causes are the result of urinary infections, which cause the loss of bladder control, called cystitis, and which are usually treated with antibiotics.

Other causes of short-term urinary incontinence are polyps, kidney stones, and, more sporadically, bladder cancer, which all generate masses of abnormal tissue and cause urge incontinence, associated with blood in the urine.

Besides the short-term causes mentioned above, there are long-term causes, such as problems caused by a lack of pelvic support. The pelvic organs hold the tissues and muscles in place and, as a result of pregnancy, there can be a weakening or stretching of these support systems, and thus the organs that receive this support move out of place. If the tissues that hold the urethra, the bladder, the uterus, or the rectum become weak, these organs descend and cause urine to leak.

There are various methods for treating urinary incontinence, from making changes in lifestyle, such as training the urinary sphincter, to physical therapy, to the placement of devices in the bladder, medicine, injections of mass-forming agents, and finally, surgery.

European Patent no. 11000182 refers to a "Laser system for nonablative treatment of mucous membranes," which includes an adapting piece placed on the side of the distal outlet of the handle, comprising a guiding element foreseen to enter into direct contact with the area to be treated, as a receptacle and guide for the adapting piece; the handle, with the adapting piece, can move lengthwise and/or rotate with respect to the guiding element.

Further devices for treatment of the vaginal wall are disclosed in documents EP 2 476 460 and WO 2011 096 006.

### Purpose of the Invention

Avoiding the surgical method, stimulating the regeneration of the pelvic tissue with the aid of laser rays generated in the corresponding device and reaching the proper area inside of the vagina to cause small burns that follow a specific pattern, stimulating the tissue adjacent to the points to be burned, causing them to retract, and then stretch and regenerate, re-establishing the pelvic floor.

### Description of the Invention

The device being recommended in the present invention is electro-mechanical, and its purpose is to direct the laser rays from a laser generator to the corresponding area or in the pattern projected onto the pelvic floor by said generator; that is to say that the generator to be used in combination with the device does not form a part of the invention. Any generator existing on the market whose features suit the treatment using the device described below and the type of urinary incontinence to be treated can be used.

The device for this purpose has been planned to allow laser rays to be directed in an angular and lengthwise direction, bringing it closer or further from the previously selected area or pattern to be treated, after entering said device inside the vagina.

In order to be able to operate on the angular directing of the laser rays emitted by the generator, the device includes a means, comprising a cylinder located inside it, which we will call the surrounding element because it surrounds another cylinder that turns with respect to the surrounding one. This is in such a manner that the combination of the surrounding outer and the inner cylinder allows the barrel of the generator located on the front base of the surrounding element to be aimed right and left, and also through the combination of fitting elements around some lengthwise arms that come out of the inner cylinder and its rear base, the barrel of the laser generator can be moved closer to or further from a reflecting mirror placed in the interior of said arms, so that the bundle of laser rays is deviated 90° towards the interior of the patient and, more specifically, towards the pelvic floor indicated by the corresponding pattern.

In order to keep the smoke given off by the vaporization of tissue from fogging up the focusing lens of the laser, a circular arrangement of holes is foreseen in the side surface of the inner cylinder, ensuring that the pumping of air reaches the interior of the device independently of its angular position.

There is also an angular arrangement of positioning studs inside the inner cylinder facing another angular arrangement of holes in the surrounding element.

The aforementioned U-shaped arms have a slight outward opening, allowing them to attach better to the inner surface of the vagina, and there is a fitting element around said arms that acts as a stop at the entrance of the vagina, limiting the introduction of the device, at the same time helping the surrounding element to slide.

Lengthwise regulation is obtained through grooves that act as marks on the surface of the U-shaped arms, to guide the operator along the length of the shot and avoid repeating it in a certain place with undesired results, such as burns not necessary for the treatment to regenerate the tissue.

Other details and characteristics will be shown over the course of the description below, which refers to the drawings that accompany this report, in which there is a graphic representation of the invention, for the purpose of illustration, not limitation.

### Description of the Figures

Below is a list of the different parts of the invention, which with the aid of the corresponding numbers allows them to be located in the figures presented below; (10) device, (11) surrounding element, (12) joint, (13) mouth, (14) air being blown in, (15) support element, (16) fitting element, (17) crown, (18) tail, (19) U-shaped support, (20) arms of (19), (21) base of the "U", (22) mirror, (23) plane of refraction, (24) ventilation holes, (25) base of the surrounding element (11), (26) grooves, (28) attachment screws, (29) hollow cylinder, (30) stud positioning, (32) side surface of the surrounding element (11), (33) side surface of the cylinder (29), (34) front base of the surrounding element (11), (35) shell.
Figure no. 1 is a side view of the device (10) that is the object of the invention, in which one can see the parts that make it up, such as the surrounding element (11), the fitting element (16), and the U-shaped supports (19) with the mirror (22).
Figure no. 2 is a cross section side view of the device (10), in A-A' according to figure no. 4.
Figure no. 3 is a cross section side view of the device (10), in B-B' according to figure no. 4.
Figure no. 4 is a frontal view of the device (10) .
Figure no. 5 is a rear view of the device (10).
Figure no. 6 is a perspective view of the device (10).

### Description of One Form of the Invention.

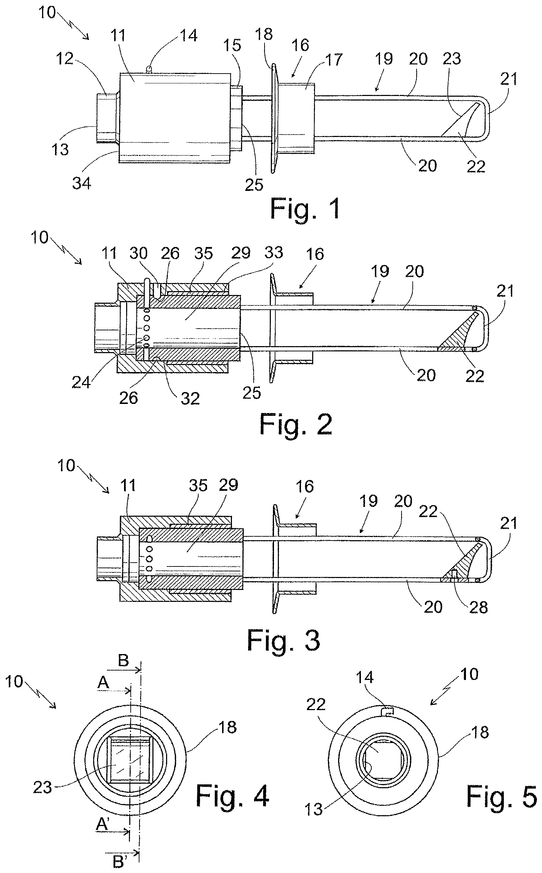

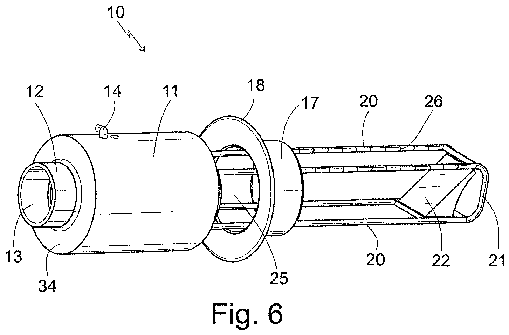

In one of the preferred forms of the invention, and as can be seen in figure no. 6, the device (10) comprises a turning mechanism comprising a surrounding element (11) with a cylindrical shape, in which a second cylinder is housed (29), see figures no. 2 and 3. In one of the bases of it, the front (34) of (11), a joint comes out (12) of the laser generator not represented in the figures, which, like a circular crown, outlines a mouth (13) into which the barrel of said generator fits, and in the opposite base (25) of the inner cylinder (29), the U-shaped supports (19) fit in, of arms (20) and base (21) of (19).

The arms (20), as can be seen in figures no. 1 and 3, are constantly in contact with the inner surface of the fitting element (16), which (16) has a crown shape (17) with a tail that sticks out perpendicularly (18), the combination of the fitting element (16) and the U-shaped supports (19) forming a means of horizontal regulation of the barrel of the generator with respect to the mirror (22), allowing it to move closer or further with respect to (22).

Inside the surrounding element (11), there is another hollow cylinder (29) that (29) turns with respect to (11), as the side surfaces (33 and 32) of the cylinders (29) and (11) are in partial direct contact, and also because of the placement of a shell (35) through the combination as is shown in figures no. 2 and 3, with grooves (26) on the side surface (32), which act as stud positioning (30), arranged in a radial manner on the surface (33) of the cylinder(29), so that the combination between the low parts (26) and the positioning pieces (30) allows both cylinders (11-29) to form a simple angular turning mechanism, to help the operator as is set out in detail below.

In the proximities of the base (21) of the supports (19) there is a mirror (22), with an inclination of (22) with respect to the arm (20) of approximately 45°, the mirror (22) being held on one of the arms (20) by way of the attachment screws (28), as can be seen in figure no. 3.

Another means of helping the operator are the grooves (27) foreseen at regular distances on the arms (20) of the supports (19), as can be seen in figure no. 6, so that the operator can check the distance of laser exposure and firing at all times, pushing the laser generator forwards and backwards, placed on the front base of the surrounding element (11) in the joint piece (15) guided by the grooves (26) that mark the distance of said generator with respect to the mirror (22).

The operating of the device (10) is, in the first place, to place it in the vagina of the patient to be treated, so that the supports (19) fit into the walls of the same, the penetration of the supports (19) being limited with the aid of the band or fitting element (16), which in turn comprises the crown (17) and the tail (18), the latter or which stops (18) at the entrance of the vagina, and which at the same time allows a linear movement of the barrel of the generator immobilised in the joint piece (12) of the surrounding element (11).

The laser rays coming from the generator located at the mouth (13) are directed at the mirror (22) and, more specifically, its polished reflective plane (23), aiming them towards the pelvic floor, for which the turning mechanism is necessary; it is made up of the cylinders (11-29) and the horizontal alignment, which along with the aid of the marks (27) on the arms (20) of the "U's"(19), allow the generator to be moved closer and further with respect to the inside of the vagina.

Treatment using the device (10) is performed with the aid of patterns, which help the so-called "sweeping phase" by previously outlining the area where the laser rays will make contact, forming a kind of rows or columns, or alternating diagonal series, so that the thermal dispersion caused by each impact of a row, column, or diagonal series doesn't join the technical dispersion caused by each impact on an adjacent row, column or diagonal series, in terms of impacts on the aforementioned area to be treated. Preferably, but not exclusively, the sweeping phase is carried out on an area shaped like a parallelogram.

The invention is defined in the claims; other embodiments being merely exemplary.

## Claims

1. A device (10) for the treatment of the walls of the vagina tissue through laser radiation, wherein the device (10) is attachable for its proximal opening to a laser generator for emitting a laser radiation towards the interior of the device (10), and wherein the laser rays directed to the device (10) can be aimed both lengthwise and at an angle at the pelvic floor in an area outlined to create a pattern of burn points aligned in rows, columns, or diagonally, **characterised in that** the device (10) comprises:
- a mechanism of angular turning made up of:
- a surrounding element (11) with a hollow cylindrical shape being located around an inner cylinder (29), and
- the inner cylinder (29) being rotatable with respect to the surrounding element (11),
and wherein:
- the surrounding element (11) further comprising an annular joint piece (12) coming out of its proximal end and having a mouth into which the barrel of the laser generator fits,
- the inner cylinder (29) further comprising means (19) to move said generator lengthwise with respect to a mirror (22), and
- the reflecting mirror (22) being located at the distal end of said means (19).

2. The device (10) according to claim 1, wherein the inner side surface of the surrounding element (11) is in part in direct contact with the outer side surface of the inner cylinder (29) and in part in contact with said outer side surface through a shell (35).

3. The device (10) according to claim 1, wherein the means to move said generator lengthwise with respect to a mirror (19) comprise U-shaped supports (19) located within the inner cylinder (29), and the supports (19) comprise arms (20) that are, during use, in constant contact with the inner surface of the vagina.

4. The device (10) according to claim 1, wherein it also has a fitting element (16) comprising an annular shape (17) fitted around the inner cylinder (29) and a tail (18) which is perpendicular to the annular part (17).

5. The device (10) according to claim 3, wherein the arms (20) are slightly divergent.

6. The device (10) according to claim 1, wherein the mirror (22) is located close to the base of the U-shaped supports, the mirror (22) being tilted with respect to the arms (20) of the U-shaped supports (19) at approximately a 45° angle, and the mirror (22) being held to the arms (20) by way of the corresponding attachment screws.

7. The device (10) according to claim 2, wherein the shell (35) is a hollow cylinder and is turned relatively with respect to the surrounding element (11), using a combination, with a set of grooves (26) that act to position the studs (30), arranged in a radial manner on the surface of the inner cylinder (29) with an angle of approximately 30°, allowing the two cylinders to form a simple angular turning mechanism to the right or left.

8. The device (10) according to claim 1, wherein there are grooves (26) for helping the operator, the grooves (26) being located lengthwise at regular distances on the arms of the U-shaped supports (19), so that the operator can check the distance of exposure and laser firing at all times, sliding the generator forwards and backwards with respect to the arms (20) of the U-shaped support (19).

9. The device (10) according to claims 1 and 7, wherein the alignment of the grooves (26) foreseen on the outer side surface of the inner cylinder (29) there is a radial alignment of holes in the surface of the surrounding element (11), and both alignments face each other.

## Patentansprüche

1. Vorrichtung (10) zum Behandeln der Wände des Vaginalgewebes durch Laserstrahlung, wobei die Vorrichtung (10) an ihrer proximalen Öffnung an einem Lasergenerator befestigt werden kann, um eine Laserstrahlung in Richtung des Innenraums der Vorrichtung (10) zu strahlen, und wobei die Laserstrahlen, die auf die Vorrichtung (10) gerichtet sind, sowohl in Längsrichtung als auch unter einem Winkel auf den Beckenboden in einen Bereich gerichtet werden können, der begrenzt ist, um ein Muster aus Verbrennungspunkten zu erzeugen, die in Reihen, Spalten oder diagonal ausgerichtet sind,
**dadurch gekennzeichnet, dass** die Vorrichtung (10) Folgendes umfasst:
- einen Mechanismus für eine Winkeldrehung, der wie folgt gebildet wird:
- ein umgebendes Element (11) mit einer Hohlzylinderform, das um einen inneren Zylinder (29) angeordnet ist, und
- wobei der innere Zylinder (29) in Bezug auf das umgebende Element (11) gedreht werden kann,
und wobei:
- das umgebende Element (11) ferner ein ringförmiges Verbindungsstück (12) umfasst, das aus dem proximalen Ende vorsteht und eine Öffnung aufweist, in die der Lauf des Lasergenerators passt,
- der innere Zylinder (29) ferner Mittel (19) umfasst, um den Generator in Bezug auf einen Spiegel (22) in Längsrichtung zu bewegen, und
- der reflektierende Spiegel (22) an dem distalen Ende der Mittel (19) angeordnet ist.

2. Vorrichtung (10) nach Anspruch 1, wobei die innere seitliche Oberfläche des umgebenden Elements (11) teilweise in direktem Kontakt mit der äußeren seitlichen Oberfläche des inneren Zylinders (29) und teilweise durch eine Hülle (35) in Kontakt mit der äußeren seitlichen Oberfläche ist.

3. Vorrichtung (10) nach Anspruch 1, wobei die Mittel zum Bewegen des Generators in Längsrichtung in Bezug auf einen Spiegel (19) U-förmige Halterungen (19) umfassen, die in dem inneren Zylinder (29) angeordnet sind, und wobei die Halterungen (19) Arme (20) umfassen, die im Einsatz mit der inneren Oberfläche der Vagina in ständigem Kontakt sind.

4. Vorrichtung (10) nach Anspruch 1, die außerdem ein Passelement (16), das eine ringförmige Form (17) umfasst, die um den inneren Zylinder (29) passt, und einen Ausläufer (18), der senkrecht zu dem ringförmigen Teil (17) ist, aufweist.

5. Vorrichtung (10) nach Anspruch 3, wobei die Arme (20) leicht auseinanderlaufen.

6. Vorrichtung (10) nach Anspruch 1, wobei der Spiegel (22) nahe an der Basis der U-förmigen Halterungen angeordnet ist, wobei der Spiegel (22) in Bezug auf die Arme (20) der U-förmigen Halterungen (19) unter einem Winkel von etwa 45° gekippt ist und wobei der Spiegel (22) an den Armen (20) mittels der entsprechenden Befestigungsschrauben gehalten wird.

7. Vorrichtung (10) nach Anspruch 2, wobei die Hülle (35) ein Hohlzylinder ist und unter Verwendung einer Kombination mit einem Satz Rillen (26), die dazu dienen, die Stifte (30) zu positionieren, wobei die Rillen auf der Oberfläche des inneren Zylinders (29) radial in einem Winkel von etwa 30° angeordnet sind, damit die zwei Zylinder einen einfachen Winkeldrehmechanismus nach rechts oder links bilden, in Bezug auf das umgebende Element (11) gedreht wird.

8. Vorrichtung (10) nach Anspruch 1, wobei es Rillen (26) gibt, um die Bedienungsperson zu unterstützen, wobei die Rillen (26) in Längsrichtung in regelmäßigen Abständen an den Armen der U-förmigen Halterungen (19) angeordnet sind, so dass die Bedienungsperson die Distanz einer Strahleneinwirkung und den Laserbeschuss zu jedem Zeitpunkt überprüfen kann, wobei der Generator in Bezug auf die Arme (20) der U-förmigen Halterung (19) vorwärts und rückwärts gleitet.

9. Vorrichtung (10) nach Anspruch 1 bis 7, wobei dann, wenn die Ausrichtung der Rillen (26) an der äußeren seitlichen Oberfläche des inneren Zylinders (29) vorgesehen ist, eine radiale Ausrichtung von Löchern in der Oberfläche des umgebenden Elements (11) vorliegt, und beide Ausrichtungen zueinander zeigen.

## Revendications

1. Dispositif (10) de traitement des parois du tissu vaginal par un rayonnement laser, où le dispositif (10) est connectable par son ouverture proximale à un générateur laser pour émettre un rayonnement laser vers l'intérieur du dispositif (10), et où les rayons laser dirigés vers le dispositif (10) peuvent être pointés dans la direction de la longueur et selon un angle au niveau du plancher pelvien dans une zone délimitée pour créer un motif des points de brûlure alignés en rangées, colonnes, ou de manière diagonale, **caractérisé en ce que** le dispositif (10) comprend :
- un mécanisme de rotation angulaire composé des éléments suivants :
- un élément enveloppant (11) qui présente une forme cylindrique creuse située autour d'un cylindre intérieur (29), et
- le cylindre intérieur (29) pouvant tourner par rapport à l'élément enveloppant (11),
et où :
- l'élément enveloppant (11) comprend en outre une partie jonction annulaire (12) sortant de son extrémité proximale et présentant une bouche dans laquelle s'ajuste le corps du générateur laser,
- le cylindre intérieur (29) comprenant en outre des moyens (19) de déplacement dudit générateur dans la direction de la longueur par rapport à un miroir (22), et
- le miroir réfléchissant (22) étant situé à l'extrémité distale desdits moyens (19).

2. Dispositif (10) selon la revendication 1, où la surface latérale intérieure de l'élément enveloppant (11) est en partie en contact direct avec la surface latérale extérieure du cylindre intérieur (29), et en partie en contact avec ladite surface latérale extérieure à travers une enveloppe (35).

3. Dispositif (10) selon la revendication 1, où les moyens de déplacement dudit générateur dans la direction de la longueur par rapport à un miroir (19) comprennent des supports en forme de U (19) situés dans le cylindre intérieur (29), et les supports (19) comprennent des bras (20) qui sont, au cours de l'utilisation, en contact permanent avec la surface intérieure du vagin.

4. Dispositif (10) selon la revendication 1, qui présente également un élément raccord (16) qui présente une forme annulaire (17) ajusté autour du cylindre intérieur (29) et une queue (18) qui est perpendiculaire à la partie annulaire (17).

5. Dispositif (10) selon la revendication 3, où les bras (20) sont légèrement divergents.

6. Dispositif (10) selon la revendication 1, où le miroir (22) se situe à proximité de la base des supports en forme de U, le miroir (22) étant incliné par rapport aux bras (20) des supports en forme de U (19) selon un angle approximativement égal à 45°, et le miroir (22) étant tenu par rapport aux bras (20) par des vis de fixation correspondantes.

7. Dispositif (10) selon la revendication 2, où l'enveloppe (35) est un cylindre creux et est tournée par rapport à l'élément enveloppant (11), en utilisant une association, avec un ensemble de rainures (26) qui agissent pour positionner les goujons (30), disposés d'une manière radiale sur la surface du cylindre intérieur (29) selon un angle approximativement égal à 30°, en permettant aux deux cylindres de former un mécanisme de rotation angulaire simple vers la gauche ou vers la droite.

8. Dispositif (10) selon la revendication 1, présentant des rainures (26) pour aider l'opérateur, les rainures (26) étant situées dans la direction de la longueur à des distances régulières sur les bras des supports en forme de U (19), de sorte que l'opérateur puisse contrôler la distance de l'exposition et le déclenchement du laser à tout moment, en faisant coulisser le générateur vers l'avant et vers l'arrière par rapport aux bras (20) du support en forme de U (19).

9. Dispositif (10) selon les revendications 1 et 7, où, sur l'alignement des rainures (26) prévues sur la surface latérale extérieure du cylindre intérieur (29), il y a un alignement radial des trous dans la surface de l'élément enveloppant (11), et les deux alignements se font face.
